# EUROPÄISCHE PATENTSCHRIFT

(11) **EP 2 948 058 B1**
(45) Veröffentlichungstag und Bekanntmachung des Hinweises auf die Patenterteilung: **21.11.2018**
(21) Anmeldenummer: 14712568.6
(22) Anmeldetag: 24.01.2014
(51) Int. Cl.: A61B 50/30, A61B 5/145, A61B 5/00, A61B 5/1455, G01N 21/80, A61B 50/00

(54) **HANDMESSGERÄT**
HAND-HELD MEASURING DEVICE
APPAREIL DE MESURE PORTATIF

(30) Priorität: 25.01.2013 DE 202013100349 U
(43) Veröffentlichungstag der Anmeldung: 02.12.2015
(73) Patentinhaber: Nawa Heilmittel GmbH, 90482 Nürnberg (DE)
(72) Erfinder: RIESINGER, Thomas, 90482 Nürnberg (DE)
(74) Vertreter: Glück Kritzenberger Patentanwälte PartGmbB
(86) Internationale Anmeldenummer: PCT/DE2014/100019
(87) Internationale Veröffentlichungsnummer: WO 2014/114290

(56) Entgegenhaltungen:
- CA-A1- 2 307 420
- DE-A1-102010 053 814
- US-A1- 2001 034 479
- US-A1- 2002 061 495
- US-A1- 2006 161 048
- US-B1- 6 847 490

## Beschreibung

### Technisches Gebiet

Die vorliegende Erfindung betrifft ein Handmessgerät zur Messung des pH-Wertes von Wunden an einem Messpunkt.

### Stand der Technik

Aus der DE 20 2010 011 934 U1 ist ein medizinisches Handmessgerät zur Messung des pH-Wertes und der Temperatur von Wunden bekannt. Das Handmessgerät weist ein Gehäuse mit wenigstens einer Anzeige zur Anzeige des jeweiligen Messwertes sowie Bedienungselemente auf. An einem Ende des Gehäuses ist eine Sonde mit einer Sondenanordnung vorgesehen. Die Sondenanordnung erzeugt dem zu messenden pH-Wert und der zu messenden Temperatur entsprechende elektrische Messsignale. Die Sonde weist einen sondenseitigen Kupplungsabschnitt auf und ist an einem gehäuseseitigen Kupplungsabschnitt abnehmbar mechanisch sowie elektrisch mit dem Gehäuse verbunden.

Das aus dem Stand der Technik bekannte Handmessgerät liefert zwar genaue und reproduzierbare Messwerte, ist aber in seinen Einsatzzeiten limitiert.

Dokument CA2307420 offenbart ebenfalls ein Handmessgerät zur Messung des pH-Wertes.

### Darstellung der Erfindung

Hier setzt die Erfindung an. Der Erfindung, wie sie in den Ansprüchen gekennzeichnet ist, liegt die Aufgabe zu Grunde, ein Handmessgerät bereitzustellen, das häufiger zu Messungen eingesetzt werden kann als die aus dem Stand der Technik bekannten Handmessgeräte.

Diese Aufgabe wird erfindungsgemäß durch das Handmessgerät gemäß Anspruch 1 gelöst. Weitere vorteilhafte Details, Aspekte und Ausgestaltungen der vorliegenden Erfindung ergeben sich aus den abhängigen Ansprüchen, der Beschreibung und dem Beispiel.

Die vorliegende Erfindung stellt ein Handmessgerät zur Messung des pH-Wertes von Wunden an einem Messpunkt zur Verfügung. Das Handmessgerät weist ein Gehäuse, eine das Gehäuse zumindest teilweise umschließende, abnehmbare Schutzhülle und eine aus zumindest zwei Elementen aufgebaute Detektions- und Auswerteeinheit auf. Die Auswerteeinheit ist zur Verarbeitung der von der Detektionseinheit detektierten Messsignale ausgebildet. Zumindest ein Element der Detektions- und Auswerteeinheit ist in oder an dem Gehäuse angeordnet und zumindest ein Element der Detektions- und Auswerteeinheit ist als Bestandteil der Schutzhülle ausgebildet.

Durch die das Gehäuse von dem Messpunkt trennende Schutzhülle wird eine Kontamination des Gehäuses durch einen Kontakt mit der Wunde vermieden. Nach der Messung kann die Schutzhülle abgenommen und durch eine andere Schutzhülle ersetzt werden. Dadurch können die Einsatzzeiten des Handmessgerätes deutlich gesteigert werden. Die gebrauchten Schutzhüllen können in einfacher Weise gereinigt und nachfolgend wiederverwendet werden. Da zumindest ein Element der Detektions- und Auswerteeinheit als Bestandteil der Schutzhülle ausgebildet ist, sind durch den Einfluss der Schutzhülle verfälschte Messungen ausgeschlossen.

Gemäß einer bevorzugten Ausführungsform der vorliegenden Erfindung ist die Schutzhülle als einmal verwendbares Produkt ausgebildet. Als Wegwerfartikel kann die Schutzhülle besonders einfach und schnell gewechselt werden und das Handmessgerät steht nach Ausstattung mit einer neuen Schutzhülle sofort wieder für eine Messung zur Verfügung. Eine nachfolgende Reinigung der Schutzhülle entfällt.

Besonders bevorzugt umschließt die Schutzhülle das Gehäuse vollständig und gegenüber der Umgebung dicht. Durch diese Ausführungsform wird eine Kontamination des Gehäuses sicher vermieden.

Bevorzugt weist das Gehäuse des Handmessgeräts einen ersten Gehäuseabschnitt und einen sich an den ersten Gehäuseabschnitt anschließenden stab- oder sondenartigen zweiten Gehäuseabschnitt auf, wobei der zweite Gehäuseabschnitt einen im Vergleich zum ersten Gehäuseabschnitt reduzierten Querschnitt aufweist. Diese Ausbildung des Handmessgerätes hat sich als besonders vorteilhaft in der Handhabung bei der Durchführung von Messungen erwiesen.

Besonders bevorzugt ist die Schutzhülle zumindest zweiteilig aus einem ersten, den ersten Gehäuseabschnitt umschließenden Schutzhüllenabschnitt sowie aus einem zweiten, den zweiten Gehäuseabschnitt umschließenden Schutzhüllenabschnitt aufgebaut. Das zumindest eine als Bestandteil der Schutzhülle ausgebildete Element der Detektions- und Auswerteeinheit ist dabei als Bestandteil des zweiten Schutzhüllenabschnitts ausgebildet.

Erfindungsgemäß handelt es sich bei dem zumindest einen als Bestandteil der Schutzhülle ausgebildeten Element der Detektions- und Auswerteeinheit um ein elektrisches Detektionselement. In oder an dem Gehäuse ist eine Auswerteeinheit zur Verarbeitung der von der Detektionseinheit detektierten Messsignale als Element der Detektions- und Auswerteeinheit vorgesehen. Das elektrische Detektionselement wird in diesem Fall mit der Schutzhülle nach der jeweiligen Messung entsorgt. Bei jeder neuen Messung steht dann ein neuwertiges Detektionselement zur Verfügung, welches eine genaue und reproduzierbare pH-Wert Messung garantiert.

Erfindungsgemäß weist die Schutzhülle einen Schaltkreis auf und die in oder an dem Gehäuse angeordnete Auswerteeinheit umfasst eine mit dem Schaltkreis zusammenwirkende erste Empfangs- und Sendeeinheit. Das elektrische Detektionselement ist als Bestandteil des Schaltkreises ausgebildet, wobei der Schaltkreis für eine drahtlose Übertragung der von dem elektrischen Detektionselement detektierten Messwerte an die Empfangs- und Sendeeinheit ausgebildet ist. Die Übertragung der den gemessenen pH-Werten entsprechenden Messsignale vom Sensorelement auf die im Gehäuse untergebrachte Messelektronik erfolgt in diesem Fall drahtlos über eine Funkstrecke. Der Schaltkreis sorgt für eine drahtlose Übertragung der vom Detektionselement erfassten Messwerte an die Empfangseinheit ausgebildet ist.

Insbesondere bevorzugt sind Ausführungsformen, gemäß denen der Schaltkreis nach Art eines RFIDs ausgebildet ist und der Schaltkreis zumindest eine Antennenstruktur sowie einen mit dem elektrischen Detektionselement zusammenwirkenden Mikroprozessor aufweist. Die Spannungsversorgung des Mikroprozessors erfolgt über ein von der Antennenstruktur empfangenes Radio- oder Funksignal.

Gemäß einer weiteren besonders bevorzugten Ausführungsform der vorliegenden Erfindung ist am oder im Gehäuse eine zweite Sende- und Empfangseinheit vorgesehen, wobei die zweite Sende- und Empfangseinheit mit einem externen Rechner über eine Datenfunkstrecke zusammenwirkt.

Die Schutzhülle besteht bevorzugt aus einem dünnen transparenten Material, welches eine preiswerte Herstellung dieser Hülle ermöglicht, besonders bevorzugt aus einem geeigneten Kunststoff.

Sämtliche beschriebenen Ausführungsformen können für sich oder in beliebiger Kombination miteinander zusammen mit dem erfindungsgemäßen Handmessgerät verwirklicht werden.

### Kurze Beschreibung der Zeichnungen

Die Erfindung soll nachfolgend anhand von Ausführungsbeispielen im Zusammenhang mit den Zeichnungen näher erläutert werden. Es wird aber ausdrücklich darauf hingewiesen, dass die Erfindung nicht auf die angegebenen Beispiele beschränkt sein soll. Es zeigen
- Fig. 1: in schematischer Darstellung eine Draufsicht auf ein Handmessgerät als nicht Teil der Erfindung;
- Fig. 2: in schematischer Darstellung eine Draufsicht auf eine weitere Ausführungsform des Handmessgeräts als nicht Teil der Erfindung;
- Fig. 3: in schematischer Darstellung eine Draufsicht auf die

Ausführungsform des Handmessgeräts gemäß der Erfindung.

### Wege zur Ausführung der Erfindung

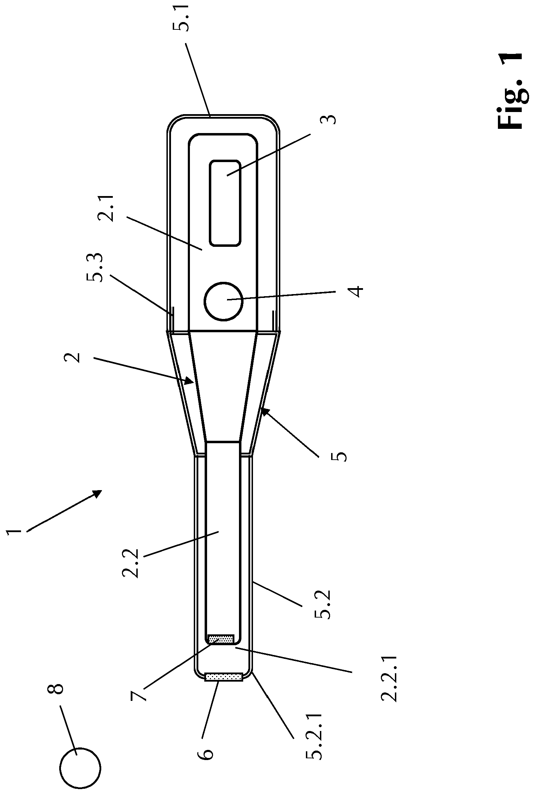

In den Figuren bezeichnet 1 ein Handmessgerät in Form eines elektronischen pH-Meters zur Messung des pH-Wertes von Wunden. Das Gehäuse 2 des Handmessgerätes 1 weist in seiner äußeren Gestaltung einen Gehäuseabschnitt 2.1 mit größerem Querschnitt und einen sich an diesen Gehäuseabschnitt stirnseitig anschließenden stab- oder sondenartigen Gehäuseabschnitt 2.2 mit reduziertem Querschnitt auf. An dem den Gehäuseabschnitt 2.1 entfernt liegenden freien Ende des Gehäuseabschnittes 2.2 ist ein für die Messung notwendiges Element einer Detektions- und Auswerteeinheit untergebracht wie dies nachstehend für verschiedene mögliche Ausführungsformen des Handmessgerätes 1 im Zusammenhang mit den Figuren 1 bis 3 erörtert wird.
Der Gehäuseabschnitt 2.1 dient u.a. zur Aufnahme einer beispielsweise einen Mikroprozessor aufweisenden Auswerteeinheit sowie zur Aufnahme einer wieder aufladbaren Batterie und eines Ladestromkreises, der beispielsweise eine kontaktlose Aufladung dieser Batterie, z.B. induktiv und/oder kapazitiv ermöglicht. Am Gehäuseabschnitt 2.1 ist weiterhin ein Display 3 vorgesehen, welches u.a. zur Anzeige des Betriebszustandes des Handmessgerätes 1 sowie des jeweiligen Messwertes (pH-Wert) dient. Ebenfalls am Gehäuseabschnitt 2.1 sind Betätigungselemente 4 vorgesehen, beispielsweise zum Aktivieren und Deaktivieren des Handmessgerätes. Das Gehäuse 2 ist dicht verschlossen, sodass ein Eindringen von Schmutz und/oder Keimen in das Innere des Gehäuses 2 ausgeschlossen ist.

Mit 5 ist in den Figuren eine Schutzhülle bezeichnet, die aus einem dünnen, flexiblen und transparenten Material besteht, beispielsweise aus einem geeigneten Kunststoff in Form einer Folie. Das Gehäuse 2 ist während der Verwendung des Messgerätes 1 gegen Verschmutzen und/oder Verkeimen verschlossen in der Schutzhülle 5 aufgenommen.

Bei der in den Figuren dargestellten Ausführungsform ist die Schutzhülle 5, die in ihrem das Gehäuse 2 umschließenden Zustand an die Form des Gehäuses 2 angepasst ist, zweiteilig ausgebildet, und zwar bestehend aus einem im Wesentlichen den Gehäuseabschnitt 2.1 aufnehmenden Schutzhüllenabschnitt 5.1 und aus einem Schutzhüllenabschnitt 5.2, der an den reduzierten Querschnitt des Gehäuseabschnittes 2.2 angepasst ebenfalls einen gegenüber dem Schutzhüllenabschnitt 5.1 reduzierten Querschnitt aufweist. Beide Schutzhüllenabschnitte 5.1 und 5.2 sind bei am Gehäuse 2 befestigter Schutzhülle 5 in dem mit 5.3 bezeichneten Verbindungsbereich dicht, aber lösbar miteinander verbunden. Dieser Verbindungsbereich befindet sich bei der dargestellten Ausführungsform etwa in der Mitte des Gehäuses 2 zwischen den einander abgewandten Enden der Gehäuseabschnitte 2.1 und 2.2.

Die Schutzhülle 5 ist als einmal verwendbarer Artikel ausgeführt, d.h. nach der jeweiligen Verwendung des Handmessgerätes 1 wird die Schutzhülle 5 von dem Gehäuse 2 entfernt und entsorgt. Durch die Schutzhülle 5 wird zum einen ein Verschmutzen und/oder Verkeimen des Gehäuses 2 bei der Verwendung des Handmessgerätes 1 vermieden. Andererseits stellt die vor jeder Verwendung des Handmessgerätes 1 neu auf das Gehäuse 2 aufgebrachte sterile Schutzhülle 5 auch sicher, dass keine Übertragung von Keimen vom Gehäuse 2 auf den jeweiligen Messbereich (z.B. Wunde) erfolgt.

Die Messung des pH-Wertes erfolgt durch die geschlossene Schutzhülle 5 bzw. durch den geschlossenen Schutzhüllenabschnitt 5.2. Dies kann auf unterschiedliche Weise realisiert werden.

Die Figur 1 zeigt in schematischer Darstellung ein Handmessgerät, bei dem die Erfassung des pH-Wertes auf optischem Wege, d.h. durch ein optisches Sensorelement erfolgt, das aber nicht Teil der vorliegenden Erfindung ist. Hierfür ist der Schutzhüllenabschnitt 5.2 an seinem stirnseitigen Ende 5.2.1, welches dem freien Ende 2.2.1 des Gehäuseabschnittes 2.2 benachbart ist, mit einem Fenster 6 versehen, das als optisches Sensorelement wirkt und dessen optische Durchlasscharakteristik sich hinsichtlich Absorption, Reflektivität und/oder Transparenz in Abhängigkeit von dem am Messort herrschenden pH-Wert verändert. Am freien Ende 2.2.1 des Gehäuseabschnittes 2.2 ist eine opto-elektronische Einheit 7 mit einem opto-elektronischen Sensor vorgesehen. Nach der jeweiligen Messung und/oder während der Messung wird das Licht einer Lichtquelle 8 durch das Fenster 6 auf die opto-elektronische Einheit 7 geleitet, die dann ein der aktuellen Durchlasscharakteristik und damit dem gemessenen pH-Wert entsprechendes elektrisches Messsignal an die Messelektronik im Gehäuse 2 liefert. Die Lichtquelle 8 kann auch an oder in dem Gehäuse 2 oder in der opto-elektronischen Einheit 7 angeordnet sein, wobei in diesem Fall das von dem Sensorelement 6 in Richtung der opto-elektronischen Einheit 7 reflektierte Licht detektiert wird.

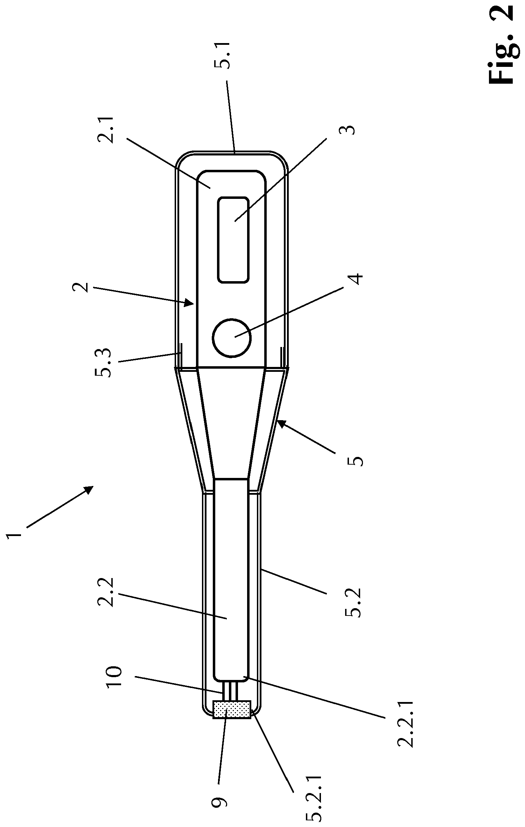

Die Figur 2 zeigt eine beispielhafte aber nicht erfindungsgemäße Ausführungsform, bei der am stirnseitigen Ende 5.2.1 des Schutzhüllenabschnitts 5.1 ein Sensorelement 9 (beispielsweise ein ISFET-Sensor) vorgesehen ist, welches ein von dem jeweils gemessenen pH-Wert abhängiges elektrisches Messsignal liefert. Das Sensorelement 9 befindet sich dabei mit seinem Sensorbereich außerhalb des Schutzhüllenabschnitts 5.2 und mit elektrischen Anschlüssen oder Elektroden innerhalb dieses Schutzhüllenabschnitts, wobei der Innenraum des Schutzhüllenabschnittes 5.2 auch im Bereich des Sensorelementes 9 hermetisch gegenüber der Umgebung verschlossen ist. Der Gehäuseabschnitt 2.2 ist bei dieser Ausführungsform an seinem Ende 2.2.1 mit elektrischen Kontakten 10, beispielsweise in Form von Kontaktstiften versehen, die im Verwendungsfall eine elektrische Verbindung mit den Kontakten des Sensorelementes 9 herstellen.

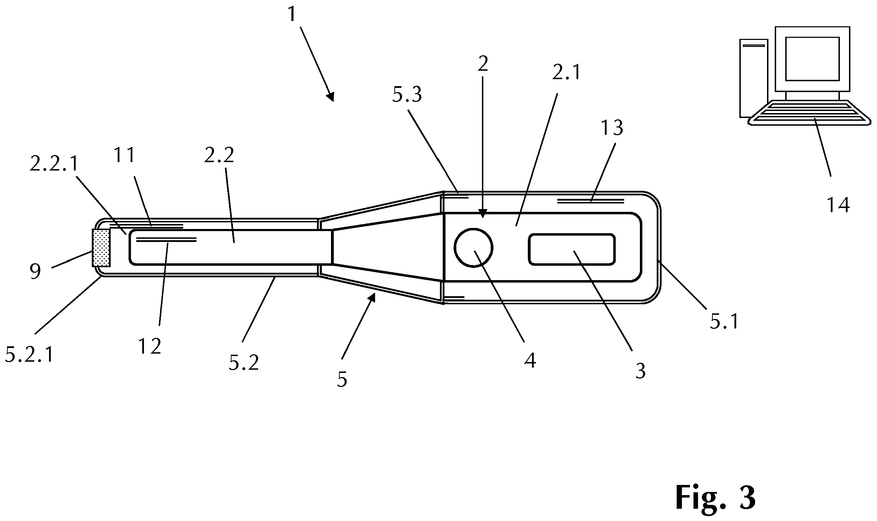

Die Figur 3 zeigt eine erfindungsgemäße Ausführungsform, bei der der Schutzhüllenabschnitt 5.2 an seinem stirnseitigen Ende 5.2.1 wiederum das Sensorelement 9 aufweist, welches aber Bestandteil eines elektrischen Schaltkreises 11 ist, der entsprechend einem RFID oder RFID-TAG mit Mikroprozessor ausgebildet ist, und zwar derart, dass die von dem Sensorelement 9 gelieferten, den gemessenen pH-Werten entsprechenden Messdaten drahtlos, d.h. über elektromagnetische Wellen an einen im Inneren des Gehäuseabschnittes 2.2 angeordnete Empfangs- und Sendeeinheit 12 übertragen werden. Der Schaltkreis 11 weist hierfür beispielsweise den von RFIDs bekannten Aufbau auf und umfasst u.a. eine Antennenstruktur, den Mikroprozessor sowie eine Spannungsversorgung für den Mikroprozessor, die aus den Funksignalen der Empfangs- und Sendeeinheit 12 gespeist wird. Abweichend von herkömmlichen, einfachen RFIDs ist der Schaltkreis 11 so ausgeführt, dass dessen Radiofrequenzsignal in Abhängigkeit von dem gemessenen pH-Wert moduliert ist.

### Bezugszeichenliste

- 1: Handmessgerät
- 2: Gehäuse
- 2.1, 2.2: Gehäuseabschnitt
- 2.2.1: stirnseitiges Ende des Gehäuseabschnittes 2.2
- 3: Display
- 4: Betätigungselement
- 5: Schutzhülle
- 5.1, 5.2: Schutzhüllenabschnitt
- 5.2.1: stirnseitiges Ende des Schutzhüllenabschnitts
- 6: optisches Sensorelement oder Fenster
- 8: Lichtquelle
- 9: elektrisches Detektionselement
- 10: Kontakt
- 11: Schaltkreis
- 12: Empfangs- und Sendeeinheit
- 13: Empfangs- und Sendeeinheit
- 14: Rechner

## Patentansprüche

1. Handmessgerät (1) zur Messung des pH-Wertes von Wunden an einem Messpunkt, wobei das Handmessgerät (1) ein Gehäuse (2), eine das Gehäuse (2) zumindest teilweise umschließende, abnehmbare Schutzhülle (5) und eine aus zumindest zwei Elementen aufgebaute Detektions- und Auswerteeinheit aufweist, wobei die Auswerteeinheit als Element der Detektions- und Auswerteeinheit zur Verarbeitung der von der Detektionseinheit detektierten Messsignale ausgebildet ist, wobei zumindest ein Element der Detektions- und Auswerteeinheit in oder an dem Gehäuse (2) angeordnet ist und zumindest ein Element (9) der Detektions- und Auswerteeinheit als Bestandteil der Schutzhülle (5) ausgebildet ist, wobei es sich bei dem zumindest einen als Bestandteil der Schutzhülle (5) ausgebildeten Element der Detektions- und Auswerteeinheit um ein elektrisches Detektionselement (9) handelt, welches dafür ausgebildet ist, ein von dem jeweils gemessenen pH-Wert abhängiges elektrisches Messsignal zu liefern, wobei die Schutzhülle (5) einen Schaltkreis (11) aufweist und die in oder an dem Gehäuse angeordnete Auswerteeinheit eine mit dem Schaltkreis (11) zusammenwirkende erste Empfangs- und Sendeeinheit (12) aufweist, wobei das elektrische Detektionselement (9) Bestandteil des Schaltkreises (11) ist, **dadurch gekennzeichnet, dass** der Schaltkreis (11) für eine drahtlose Übertragung der von dem elektrischen Detektionselement (9) detektierten Messwerte an die Empfangs- und Sendeeinheit (12) ausgebildet ist.

2. Handmessgerät nach Anspruch 1, **dadurch gekennzeichnet, dass** die Schutzhülle (5) das Gehäuse (2) vollständig und gegenüber der Umgebung dicht verschließt.

3. Handmessgerät nach einem der vorhergehenden Ansprüche, **dadurch gekennzeichnet, dass** das Gehäuse (2) aus einem ersten Gehäuseabschnitt (2.1) und einem sich an den ersten Gehäuseabschnitt (2.1) anschließenden stab- oder sondenartigen zweiten Gehäuseabschnitt (2.2) besteht, wobei der zweite Gehäuseabschnitt (2.2) einen im Vergleich zum ersten Gehäuseabschnitt (2.1) reduzierten Querschnitt aufweist.

4. Handmessgerät nach Anspruch 3, **dadurch gekennzeichnet, dass** die Schutzhülle (5) zumindest zweiteilig aus einem ersten, den ersten Gehäuseabschnitt (2.1) umschließenden Schutzhüllenabschnitt (5.1) sowie aus einem zweiten, den zweiten Gehäuseabschnitt (2.2) umschließenden Schutzhüllenabschnitt (5.2) ausgebildet ist, wobei das zumindest eine als Bestandteil der Schutzhülle (5) ausgebildete Element (6, 9) der Detektions- und Auswerteeinheit als Bestandteil des zweiten Schutzhüllenabschnitts (5.2) ausgebildet ist.

5. Handmessgerät nach einem der vorhergehenden Ansprüche, **dadurch gekennzeichnet, dass** der Schaltkreis (11) nach Art eines RFIDs ausgebildet ist und zumindest eine Antennenstruktur sowie einen mit dem elektrischen Detektionselement (9) zusammenwirkenden Mikroprozessor aufweist, und dass die Spannungsversorgung des Mikroprozessors über ein von der Antennenstruktur empfangbares Radio- oder Funksignal erfolgt.

6. Handmessgerät nach einem der vorhergehenden Ansprüche, **dadurch gekennzeichnet, dass** an oder in dem Gehäuse (2) eine zweite Sende- und Empfangseinheit (13) vorgesehen ist, wobei die zweite Sende- und Empfangseinheit (13) mit einem externen Rechner (14) über eine Datenfunkstrecke zusammenwirkt.

## Claims

1. Hand-held measuring device (1) for measuring the pH value of wounds at a measuring point, wherein the hand-held measuring device (1) comprises a housing (2), a detachable protective cover (5) at least partially surrounding the housing (2) and a detection and evaluation unit made up of at least two elements, wherein the evaluation unit is configured as element of the detection and evaluation unit and is designed for the processing of the measuring signals detected by the detection unit, wherein at least one element of the detection and evaluation unit is arranged in or on the housing (2) and at least one element (9) of the detection and evaluation unit is designed as a component part of the protective cover (5), wherein the at least one element of the detection and evaluation unit designed as component part of the protective cover (5) is an electrical detection element (9) which is designed to provide an electrical signal dependent on the respectively measured pH value, wherein the protective cover (5) comprises a circuit (11) and the evaluation unit arranged in or on the housing comprises a receiving and transmitting unit (12) which interacts with the circuit (11), wherein the electrical detection element (9) is part of the circuit (11), **characterised in that** the circuit (11) is designed for the wireless transmission of measuring values detected by the detection unit (9) to the receiving and transmitting unit (12).

2. Hand-held measuring device according to claim 1 **characterised in that** the protective cover (5) seals the housing (2) completely and with respect to the surroundings in a tight manner.

3. Hand-held measuring device according to one of the preceding claims **characterised in that** the housing (2) consists of a first housing section (2.1) and a rod or probe- like second housing section (2.2) adjoining the first housing section (2.1), wherein the second housing section (2.2) comprises a reduced cross-section in comparison with the first housing section (2.1).

4. Hand-held measuring device according to claim 3 **characterised in that** the protective cover (5) is formed in at least two parts of a first protective cover section (5.1) surrounding the first housing section (2.1) and also a second protective cover section (5.2) surrounding the second housing section (2.2), wherein the at least one element (6, 9) of the detection and evaluation unit designed as a component part of the protective cover (5) is designed as a component part of the second protective cover section (5.2).

5. Hand-held measuring device according to one of the preceding claims **characterised in that** the circuit (11) is designed in the form of an RFID and comprises at least one antenna structure and a microprocessor working together with the electrical detection element (9) and **in that** the voltage supply to the microprocessor takes place via a radio or broadcast signal which can be received by the antenna structure.

6. Hand-held measuring device according to one of the preceding claims **characterised in that** on or in the housing (2) a second transmitting and receiving unit (13) is provided, wherein the second transmitting and receiving unit (13) works together with an external computer (14) via a data transmission path.

## Revendications

1. Dispositif manuel de mesure (1), destiné à mesurer la valeur pH de plaies sur un point de mesure, le dispositif manuel de mesure (1) comportant un boîtier (2), une gaine protectrice (5) amovible, entourant au moins partiellement le boîtier (2) et un module de détection et d'évaluation assemblé à partir d'au moins deux éléments, le module d'évaluation étant un élément du module de détection et d'évaluation et étant conçu pour traiter les signaux de mesure détectés par le module de détection, au moins un élément (9) du module de détection et d'évaluation étant placé dans ou sur le boîtier (2) et au moins un élément (9) du module de détection et d'évaluation étant conçu sous la forme de partie intégrante de la gaine protectrice (5), l'au moins un élément du module de détection et d'évaluation conçu en tant que partie intégrante de la gaine protectrice (5) étant un élément de détection électrique (9), lequel est conçu pour fournir un signal de mesure électrique dépendant de la valeur pH mesurée en question, la gaine protectrice (5) comportant un circuit (11) et le module d'évaluation placé dans ou sur le boîtier comportant un premier module récepteur et émetteur (12) coopérant avec le circuit (11), l'élément de détection électrique (9) étant une partie intégrante du circuit (11), **caractérisé en ce que** le circuit (11) est conçu pour une transmission sans fil au module récepteur et émetteur (12) des valeurs de mesure détectées par l'élément de détection électrique (9).

2. Dispositif manuel de mesure selon la revendication 1, **caractérisé en ce que** la gaine protectrice (5) ferme totalement le boîtier (2) et par rapport à l'environnement étanchement.

3. Dispositif manuel de mesure selon l'une quelconque des revendications précédentes, **caractérisé en ce que** le boîtier (2) est constitué d'un premier segment de boîtier (2.1) et d'un deuxième segment de boîtier (2.2) du type d'une tige ou d'une sonde, se raccordant sur le premier segment de boîtier (2.1), le deuxième segment de boîtier (2.2) présentant une section transversale réduite en comparaison du premier segment de boîtier (2.1).

4. Dispositif manuel de mesure selon la revendication 3, **caractérisé en ce que** la gaine protectrice (5) est conçue au moins en deux parties, d'un premier segment de gaine protectrice (5.1), entourant le premier segment de boîtier (2.1), ainsi que d'un deuxième segment de gaine protectrice (5.2) entourant le deuxième segment de boîtier (2.2), l'au moins un élément (6, 9) du module de détection et d'évaluation conçu en tant que partie intégrante de la gaine protectrice (5) étant conçu en tant que partie intégrante du deuxième segment de gaine protectrice (5.2).

5. Dispositif manuel de mesure selon l'une quelconque des revendications précédentes, **caractérisé en ce que** le circuit (11) est conçu à la manière d'une RFID et comporte au moins une structure d'antenne, ainsi qu'un micro-processeur coopérant avec l'élément de détection électrique (9) et **en ce que** l'alimentation en voltage du micro-processeur s'effectue par l'intermédiaire d'un signal radio ou radioélectrique, susceptible d'être réceptionné par la structure d'antenne.

6. Dispositif manuel de mesure selon l'une quelconque des revendications précédentes, **caractérisé en ce que** sur ou dans le boîtier (2) est prévu un deuxième module émetteur et récepteur (13), le deuxième module émetteur et récepteur (13) coopérant avec un ordinateur externe (14) par l'intermédiaire d'un trajet radioélectrique de données.
